(19) Europäisches Patentamt
European Patent Office
Office européen des brevets

(11) Veröffentlichungsnummer: **0 297 620 B1**

(12) **EUROPÄISCHE PATENTSCHRIFT**

(45) Veröffentlichungstag der Patentschrift: **30.11.94**

(51) Int. Cl.$^5$: **C07D 209/52**, C07D 209/42, C07C 229/28, C07C 235/26

(21) Anmeldenummer: **88110612.4**

(22) Anmeldetag: **02.07.88**

# (54) Verfahren zur Herstellung bicyclischer Aminocarbonsäuren, Zwischenprodukte dieses Verfahrens und deren Verwendung.

(30) Priorität: **03.07.87 DE 3722007**

(43) Veröffentlichungstag der Anmeldung:
**04.01.89 Patentblatt 89/01**

(45) Bekanntmachung des Hinweises auf die Patenterteilung:
**30.11.94 Patentblatt 94/48**

(84) Benannte Vertragsstaaten:
**AT BE CH DE ES FR GB GR IT LI LU NL SE**

(56) Entgegenhaltungen:
**EP-A- 0 079 022**
**EP-A- 0 084 164**
**EP-A- 0 132 580**
**EP-A- 0 173 199**
**WO-A-86/00896**

**TETRAHEDRON LETTERS Band 26, Nr. 12, 1985, Seiten1519-1522, GB; M. MORI et al.: "Reaction of alpha-haloester having internal-double bond with the low-valent metal complex"**

**TETRAHEDRON LETTERS Band 28, Nr. 13, 1987, Seiten1413-1416, GB; D.H.R. BARTON et al.: "Synthesis of 2S, 3aS, 7aS- and of 2S,3aR, 7aR-perhydroindole-2-carboxylic acid derivatives from L-aspartic acid"**

**JOURNAL OF ORGANIC CHEMISTRY Band 50, Nr. 26, 1985, Seiten5620-5627, US; A. PADWA et al.: "Synthesis of the pyrrolidine ring system byradical cyclization"**

(73) Patentinhaber: **HOECHST AKTIENGESELLSCHAFT**

**D-65926 Frankfurt (DE)**

(72) Erfinder: **Urbach, Hansjörg, Dr.**
**Le Lavandoustrasse 41**
**D-6242 Kronberg/Taunus (DE)**
Erfinder: **Henning, Rainer, Dr.**
**Rotenhofstrasse 31**
**D-6234 Hattersheim am Main (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Beschreibung**

Acylderivate der Octahydroindol-2-carbonsäure, der Octahydrocyclopenta[b]pyrrol-2-carbonsäure bzw. der Decahydrocyclohepta[b]pyrrol-2-carbonsäure sind beispielsweise bekannt aus EP-A-79022, EP-A-50800, EP-A-84164, EP-A-111873, EP-A-37231, US-Patent 4 350 704 oder US-Patent 4 587 258. Viele dieser Verbindungen zeigen eine bemerkenswerte biologische Aktivität. Sie hemmen beispielsweise hochwirksam das Angiotensin-Converting-Enzyme oder zeichnen sich durch eine nootrope Wirkung aus.

Verbindungen der Formel I

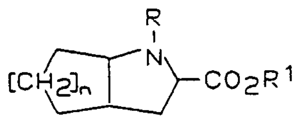

(I)

worin n = 1-3, R Wasserstoff oder einen Acylrest und $R^1$ Wasserstoff, eine veresternde Gruppe oder eine andere Carboxylschutzgruppe bedeuten, spielen eine Schlüsselrolle bei der Synthese der eingangs genannten Acylderivate.

Oft ist es vorteilhaft, wenn das Kohlenstoffatom in Position 2 des bicyclischen Ringsystems dieser Wirkstoffe eine bestimmte absolute Konfiguration, vorzugsweise die S-Konfiguration aufweist. Man geht daher bei ihrer Synthese bevorzugt von Zwischenprodukten der Formel I aus, welche diese gewünschte Konfiguration am C-2 bereits aufweisen.

Bei einigen der bekannten Herstellungsverfahren für Verbindungen der Formel I war eine Racematspaltung unverzichtbar, wollte man zu Verbindungen mit einer definierten Konfiguration am C-2 gelangen.

Aus Tetrahedron Letters 1987 1413-1416 ist ein Verfahren bekannt, mit dessen Hilfe man ausgehend von L-Asparaginsäure in einer insgesamt 12-stufigen Synthese zu optisch einheitlichen Octahydroindol-Derivaten mit einer definierten Konfiguration an C-2 gelangt.

In Tetrahedron Letters (1985) 1519-1522 wird die Cyclisierung von $\alpha$-Haloestern, die eine interne Doppelbindung aufweisen,unter Verwendung katalytischer Mengen eines niedrigvalenten Metallkomplexes beschrieben.

Aus J. Org. Chem. (1985) 5620-5627 ist die radikalische Cyclisierung von Pyrrolidinverbindungen unter Verwendung von Tri-n-butylinhydrid und Azoisobuttersäurenitril bekannt.

Es wurde nun gefunden, daß sich entsprechend substituierte Serinderivate durch Cyclisierung in optisch einheitliche Verbindungen der Formel I mit der gewünschten Konfiguration an C-2 überführen lassen, ohne daß auf irgend einer Stufe dieses neuen Verfahrens eine Racematspaltung notwendig wäre.

Die Erfindung betrifft ein Verfahren zur Herstellung von Verbindungen der Formel I
in welcher
n = 1, 2 oder 3
R ($C_1$-$C_{14}$)-Acyl und
$R^1$ ($C_1$-$C_6$)-Alkyl, ($C_3$-$C_7$)-Cycloalkyl, ($C_7$-$C_{11}$)-Aralkyl oder eine andere Carboxylschutzgruppe bedeuten, wobei die Wasserstoffatome an den Brückenkopf-Kohlenstoffatomen 3a und (5+n)a vorzugsweise cis-konfiguriert sind, das dadurch gekennzeichnet ist, daß man Verbindungen der Formel II

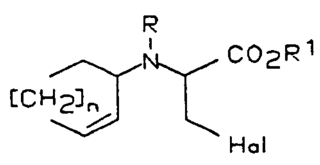

(II)

in welcher n, R und $R^1$ wie oben definiert sind und Hal Chlor, Brom oder Iod bedeutet, in Gegenwart von Trialkylstannan und gegebenenfalls eines Radikalinitiators radikalisch cyclisiert.

Wie folgendes Schema zeigt, gelangt man mit Hilfe der erfindungsgemäßen Verfahren ausgehend von beispielsweise 3-Bromcyclopenten (V) und L-Serin (III) in einer insgesamt nur 7-stufigen Synthese zu den optisch einheitlichen Diastereomeren Ia und Ib, die Homologe der oben erwähnten Octahydroindol-Derivate sind. Wenn man das Verfahren über die Stufe des L-Serinbenzylesters durchführt, verringert sich sogar dessen Stufenzahl um eine Stufe auf insgesamt 6 Stufen.

**Schema:**

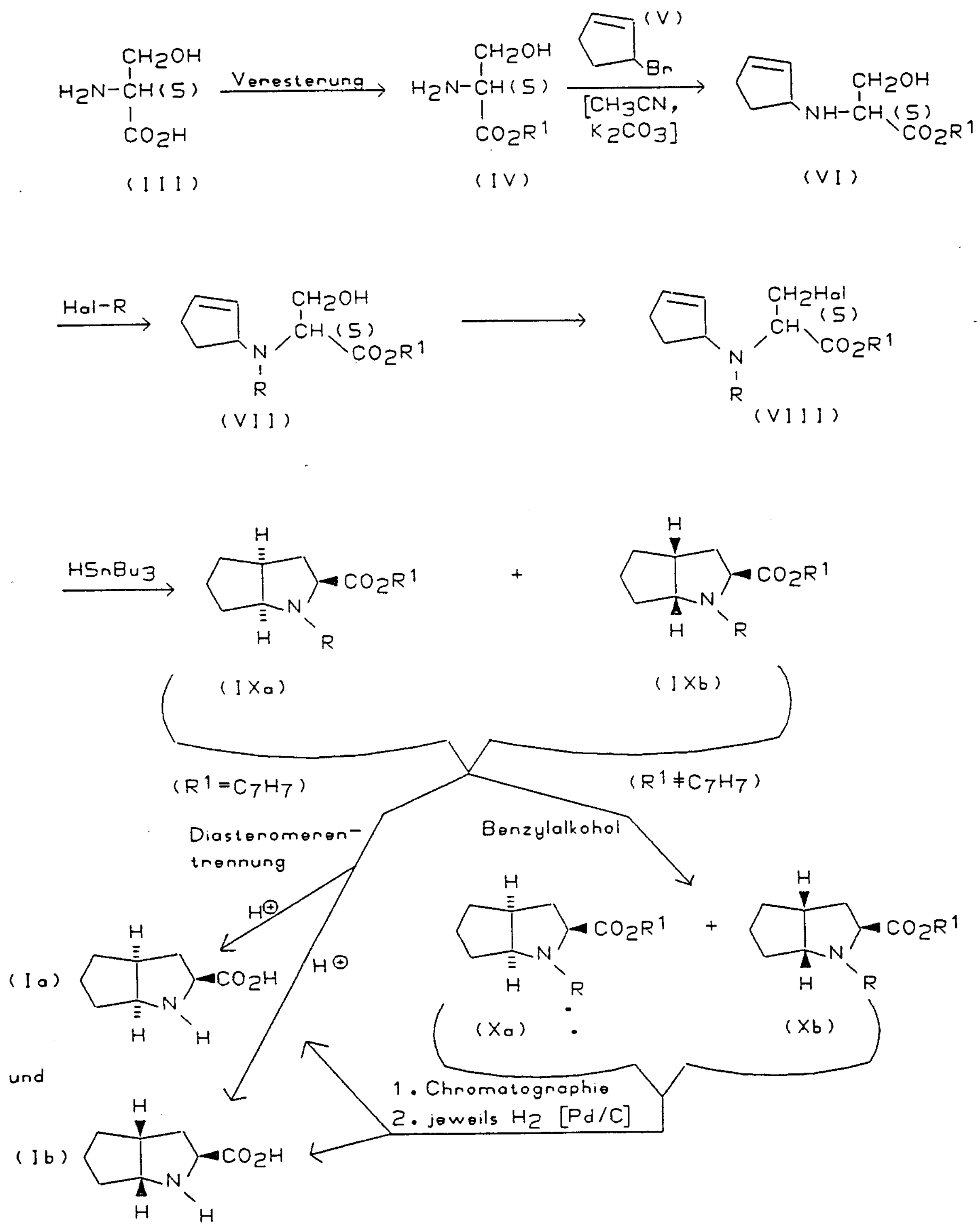


Das Kohlenstoffatom in Position 2 des bicyclischen Ringsystems der Verbindungen der Formeln I und II kann sowohl die R- als auch die S-Konfiguration aufweisen; bevorzugt ist die S-Konfiguration.

R ist vorzugsweise ($C_1$-$C_6$)-Alkanoyl, ($C_6$-$C_{10}$)-Aryl-($C_1$-$C_4$)-alkanoyl, ($C_6$-$C_{10}$)-Aroyl, ($C_1$-$C_6$)-Alkoxycarbonyl oder ($C_7$-$C_{11}$)-Aralkyloxycarbonyl, insbesondere aber ($C_1$-$C_4$)-Alkanoyl, wie Acetyl oder Propionyl, Benzoyl oder substituiertes Benzoyl, wie beispielsweise Halogenbenzoyl, Methoxybenzoyl, Dimethoxybenzoyl oder Nitrobenzoyl.

Des weiteren kann R, falls von den vorstehenden Definitionen nicht schon umfaßt, für eine in der Peptidchemie übliche Aminschutzgruppe vom Urethantyp stehen (vgl. z.B. Hubbuch, Kontakte Merck 3/79,

14-22). Schutzgruppen vom Urethantyp sind beispielsweise Pyoc, Fmoc, Tcboc, Z, Boc, Ddz, Bpoc, Adoc, Msc, Moc, $Z(NO_2)$, $Z(Hal_n)$, Dobz, Iboc, Adpoc, Mboc und 1,4-Dimethyl-pyridyl-oxycarbonyl.

$R^1$ ist vorzugsweise ($C_1$-$C_4$)-Alkyl, wie z.B. Methyl, Ethyl oder tert.-Butyl, oder ($C_7$-$C_{11}$)-Aralkyl, wie beispielsweise Benzyl.

Weiterhin kann $R^1$, falls von den vorstehenden Definitionen nicht schon umfaßt, für eine in der Peptidchemie übliche Carboxylschutzgruppe stehen (vgl. z.B. oben genannten Artikel von Hubbuch), beispielsweise die schon erwähnten Alkylreste oder Benzyl. Weiter sind modifizierte Benzylreste, wie p-Nitrobenzyl, p-Methoxybenzyl, p-Brombenzyl, p-Chlorbenzyl und Reste wie 4-Picolyl oder Benzoylmethyl geeignet.

Unter Alkyl wird im vorstehenden und im folgenden geradkettiges oder verzweigtes Alkyl verstanden. Entsprechendes gilt für davon abgeleitete Reste wie z.B. Alkanoyl und Aralkyl. Niederalkyl weist vorzugsweise bis zu 6 C-Atome auf. ($C_6$-$C_{10}$)-Aryl ist beispielsweise Phenyl oder Naphthyl; bevorzugt ist Phenyl. Entsprechendes gilt für davon abgeleitete Reste wie z.B. Aroyl und Aralkyl.

Die radikalische Cyclisierung kann mit Trialkylstannanen, wie z. B. Tri-n-butlyzinnhydrid in einem geeigneten Lösungsmittel, gegebenenfalls in Gegenwart eines Radikalinitiators, durchgeführt werden.

Geeignete Initiatoren sind beispielsweise organische Peroxide, wie tert.-Butylperoxid, substituierte Azobisalkansäurenitrile (AIBN), wie z.B. 2,2'-Azoisobuttersäurenitril (AIBN), Mercaptane und Stannane, bevorzugt ist AIBN.

Die radikalische Cyclisierung kann in einem geeigneten Lösungsmittel zwischen -20 °C und 120 °C, vorzugsweise zwischen 0 °C und dem Siedepunkt des Reaktionsgemisches, insbesonderer bei Siedetemperatur durchgeführt werden. Als Lösungsmittel kommen dabei insbesondere aprotische Lösungsmittel wie Benzol, Toluol oder Xylol in Frage.

Des weiteren kann die radikalische Cyclisierung in einem geeigneten dipolar aprotischen Lösungsmittel zwischen -20 °C und dem Siedepunkt des Reaktonsgemisches vorzugsweise zwischen 10 und 50 °C durchgeführt werden. Geeignete dipolar aprotische Lösungsmittel sind beispielsweise Ether, wie Diethylether, Tetrahydrofuran und Dioxan.

Man stellt die Verbindungen der Formel II her ausgehend von Cycloalkenylbromiden der Formel XI,

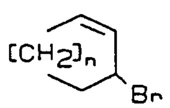 (XI)

worin n = 1, 2 oder 3 ist. Diese werden mit Serinderivaten der Formel IV, in der $R^1$ wie oben definiert ist, vorzugsweise ($C_1$-$C_6$)-Alkyl oder ($C_7$-$C_{11}$)-Aralkyl, wie Methyl oder Benzyl bedeutet und die R- oder S-, vorzugsweise S-konfiguriert sind, in Gegenwart einer Base wie $K_2CO_3$ in einem dipolar aprotischen Lösungsmittel wie Acetonitril zwischen 0 °C und dem Siedepunkt des Reaktionsgemisches, vorzugsweise bei Raumtemperatur zu Verbindungen der Formel XII

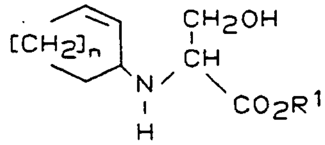 (XII)

worin n und $R^1$ wie oben definiert sind, umgesetzt.

Da die Verbindung der Formel XII als Diastereomerengemisch vorliegen kann, kann dieses gegebenenfalls über Salzbildung und fraktionierte Kristallisation oder durch Chromatographie in die reinen Diastereomeren getrennt werden. Werden die reinen Diastereomeren in die Reaktionsfolge eingesetzt, so entfällt auf einer späteren Stufe die Diastereomerentrennung, was sich dann günstig auf die Gesamtausbeute auswirkt.

Verbindungen der Formel XII werden nun zu Verbindungen der Formel XIII acyliert,

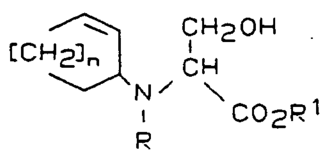 (XIII)

worin n, R und $R^1$ wie oben definiert sind. Die Acylierung führt man zweckmäßig vorzugsweise in Gegenwart einer Base in einem dipolar aprotischen Lösungsmittel wie Aceton zwischen -20 °C und dem Siedepunkt des Reaktionsgemisches, vorzugsweise bei Raumtemperatur durch. Als Acylierungsmittel kommen beispielsweise die Chloride der Formel RCl oder die Anhydride der Formel $R_2O$ in Frage. Geeignete Basen sind tert. Amine wie Triethylamin und anorganische Basen wie $K_2CO_3$.

Die Umsetzung der Verbindungen der Formel XIII zu Verbindungen der Formel II, worin n, R, $R^1$ und Hal wie oben definiert sind, erfolgt zweckmäßigerweise so, daß man die Hydroxyfunktion der Verbindungen der Formel XIII durch eine Abgangsgruppe ersetzt. So lassen sich nach bekannten Verfahren beispielsweise die entsprechenden Tosylate, Mesylate oder Triflate herstellen, die anschließend mit Chlorid, Bromid oder Iodid nucleophil in die Verbindungen der Formel II überführt werden können.

Chlor kann aber auch direkt eingeführt werden, z.B. durch Umsetzung der Verbindungen der Formel XIII mit $PCl_5$, Brom z.B. durch Umsetzung mit $PBr_3$. Die Iodverbindung der Formel II wird zweckmäßigerweise mit Triphenylphosphin und Iod in Gegenwart von Imidazol vorzugsweise bei Raumtemperatur in einem aprotischen unpolaren Lösungsmittel wie z.B. Benzol oder Toluol aus Verbindungen der Formel XIII hergestellt.

Die Erfindung betrifft auch die Zwischenprodukte der Formel IIa,

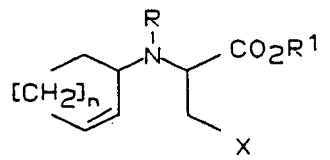

(IIa)

in welcher

n = 1, 2 oder 3,

X = Hydroxy, Chlor, Brom oder Iod,

R = Wasserstoff oder ($C_1$-$C_{14}$)-Acyl und

$R^1$ = ($C_1$-$C_6$)-Alkyl, ($C_3$-$C_7$)-Cycloalkyl, ($C_7$-$C_{11}$)-Aralkyl oder eine in der Peptidchemie übliche Carboxylschutzgruppe bedeuten.

Die nachstehenden Beispiele dienen zur Erläuterung der Erfindung, ohne daß diese darauf beschränkt wäre.

**Beispiel 1**

N-(2-Cyclopenten-1-yl)-L-serinmethylester

Zu 24,5 g L-Serinmethylester-hydrochlorid in 200 ml trockenem Acetonitril werden 48 g festes $K_2CO_3$ gegeben. Zu dieser Mischung wird 23,2 g Cyclopentenylbromid in Tetrachlorkohlenstoff bei Eiskühlung gegeben. Man läßt auf Raumtemperatur kommen und rührt 2 Stunden bei dieser Temperatur. Nach dem Absaugen des Feststoffes wird das Filtrat eingeengt und der Rückstand über Kieselgel mit $CH_2Cl_2$ als Elutionsmittel chromatographiert.
Ausbeute: 7,6 g; Fp. 113-126 °C
$[\alpha]_D^{20}$ : -30,5° (c = 1,13; $CH_3OH$)

**Beispiel 2**

N-(2-(1S)-Cyclopenten-1-yl)-L-serinmethylester

Das Diastereome rengemisch aus Beispiel 1 wird mit ethanolischer HCl in Essigester in das Hydrochlorid überführt (Fp. 150-160 °C, $[\alpha]_D^{20}$ = 10,9° (c = 0,96; $CH_3OH$) und anschließend mehrere Male aus trockenem Acetonitril umkristallisiert.
Die S,S-Verbindung (94 %ig, als HCl-Salz) hat einen Drehwert von $[\alpha]_D^{20}$ = -67,5° (c = 0,85; $CH_3OH$); Fp. 180 °C
Die freie Base wird aus dem Hydrochlorid mit wäßriger $K_2CO_3$-Lösung freigesetzt:
$[\alpha]_D^{20}$ = -111,7° (c = 0,86; $CH_3OH$)

**Beispiel 3**

N-(2-(1R)-Cyclopenten-1-yl)-L-serinmethylester

Analog Beispiel 2 wird die (R,S)-Verbindung als HCl-Salz durch Umkristallisation des Hydrochlorids in trockenen Acetonitril, Essigester und $CH_2Cl_2$ erhalten (ca. 85 %ig). Fp. 152-154 °C; $[\alpha]_D^{20}$ = +82,78° (c = 0,61; $CH_3OH$).
$[\alpha]_D^{20}$ der freien Base: +38,3° (c = 0,88; $CH_3OH$)

**Beispiel 4**

N-Benzyloxycarbonyl-N-(2-(1R,S)-cyclopenten-1-yl)-L-serinmethylester

10,5 g Methylester aus Beispiel 1 werden in 164 ml gesättigter wäßriger $NaHCO_3$-Lösung suspendiert. Dazu werden bei Raumtemperatur 11,24 ml Chlorameisensäurebenzylester gegeben. Nach 2 Stunden Rühren wird mit Essigester extrahiert. Die organische Phase wird nacheinander mit 2N wäßriger HCl, halbgesättigter wäßriger $NaHCO_3$-Lösung, Wasser und gesättigter wäßriger NaCl-Lösung gewaschen. Nach dem Trocknen wird eingeengt.
Der Rückstand wird über Kieselgel mit $CH_2Cl_2$/Essigester 95:5 chromatographiert.
Ausbeute: 14,4 g, $[\alpha]_D^{20}$ = -65° (c = 1; $CH_3OH$)

**Beispiel 5**

2-(S)-[N-Benzyloxycarbonyl-N-(2-(1R,S)-cyclopenten-1-yl-amino)]-3-iod-propionsäuremethylester

0,412 g Triphenylphosphin und 0,107 g Imidazol werden in 7 ml trockenem Benzol vorgelegt. Dazu werden 0,346 g Iod in 3 ml trockenem Benzol bei Raumtemperatur zugetropft. Es wird nach Ausfallen eines gelben Niederschlages 10 Minuten nachgerührt. Danach werden bei Raumtemperatur und unter Lichtschutz 0,319 g des Alkohols aus Beispiel 4 in 2 ml trockenem Benzol zugetropft. Es wird 3 Stunden bei Raumtemperatur gerührt. Danach wird die Mischung auf Ether/Wasser gegossen. Die Etherlösung wird mit Wasser gewaschen, getrocknet und einrotiert. Der Rückstand wird über Kieselgel mit Cyclohexan/Essigester 9:1 chromatographiert.
Ausbeute: 0,2 g Öl; $R_f$: 0,65 ($SiO_2$; $CH_2Cl_2$/Essigester 95:5; $I_2$)

**Beispiel 6**

N-Benzyloxycarbonyl-(1S,3S,5S)-2-azabicyclo[3.3.0]octan-3-carbonsäuremethylester und N-Benzyloxycarbonyl-(1R,3S,5R)-2-azabicyclo-(3.3.0]-octan-3-carbonsäuremethylester

3,25 g Iodverbindung aus Beispiel 5, 521 mg Azobisisobutyronitril (AIBN) und 2,31 g Tri-n-butylzinnhydrid werden in 260 ml trockenem Benzol gelöst. Es wird 4 Stunden am Rückfluß unter Stickstoff gekocht. Danach wird einrotiert und der Rückstand in Ether aufgenommen. Die Etherlösung wird 30 Minuten mit 10 %iger wäßriger KF-Lösung gerührt; nach Filtration wird die Etherlösung getrocknet und einrotiert.
Der Rückstand wird über Kieselgel chromatographiert mit Cyclohexan/Essigester 4:1.
Ausbeute: 2,0 g $(\alpha]_D^{20}$ = -40,5° (c = 1,035; $CH_3OH$)

**Beispiel 7**

N-Benzyloxycarbonyl-(1R,3S,5R)-2-azabicyclo-(3.3.0]-octan-3-carbonsäurebenzylester

1 g Diastereomerengemisch aus Beispiel 6 werden in 10 ml Benzylalkohol gelöst, 0,35 ml Titantetraisopropylat zugetropft und im Ölpumpenvakuum 4 Stunden bei 90 °C gerührt. Anschließend werden weitere 1,6 ml Titantetraisopropylat zugetropft und 6 Stunden bei 90 °C in Ölpumpenvakuum gerührt. Dann wird der Benzylalkohol im Vakuum entfernt und der Rückstand in Ether aufgenommen, der Ether mit 2N wäßriger HCl, dann mit gesättigter wäßriger $NaHCO_3$-Lösung und nach dem Absaugen des Niederschlages mit gesättigter wäßriger NaCl-Lösung gewaschen. Nach dem Trocknen wird einrotiert. Der Rückstand wird über Kieselgel mit Cyclohexan/Essigester 9:1 chromatographiert.
Das zuerst eluierte Produkt ist die (1R,3S,5R)-Verbindung (cis, exo-Konfiguration).

Ausbeute: 422 mg $[\alpha]_D^{20}$ = -101,6° (c = 0,82; $CH_3OH$)

**Beispiel 8**

N-Benzyloxycarbonyl-(1S,3S,5S)-2-azabicyclo-[3.3.0]-octan-3-carbonsäurebenzylester

Das nach der cis,exo-Verbindung eluierte Produkt aus Beispiel 7 ist die (1S,3S,5S)-Verbindung (cis, endo-Konfiguration).
Ausbeute: 553 mg Öl (aus Ansatz des Beispiels 7)
$[\alpha]_D^{20}$ = -2,8° (c = 1,1; $CH_3OH$)

**Beispiel 9**

(1R,3S,5R)-2-Azabicyclo-[3.3.0]-octan-3-carbonsäure

400 mg des Endproduktes aus Beispiel 7 werden in 10 ml Ethanol gelöst, dazu 50 mg Pd/C (10 %ig) gegeben und 6 Stunden hydriert.
Nach dem Absaugen des Katalysators wird eingeengt und der Rückstand mit Essigester verrührt.
Ausbeute: 90 mg; Fp. 220-225 °C
$[\alpha]_D^{20}$ = -48,4° (c = 0,37; $CH_3OH$)

**Beispiel 10**

(1S,3S,5S)-2-Azabicyclo-[3.3.0]-octan-3-carbonsäure

Analog Beispiel 9 werden 500 mg aus Beispiel 8 umgesetzt.
Ausbeute: 186 mg; Fp. 235-238 °C
$[\alpha]_D^{20}$ = -53° (c = 0,52; $CH_3OH$)

**Patentansprüche**
**Patentansprüche für folgende Vertragsstaaten : AT, BE, CH, DE, FR, GB, GR, IT, LI, LU, NL, SE**

**1.** Verfahren zur Herstellung einer Verbindung der Formel I

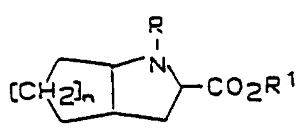

(I)

in welcher
n = 1, 2 oder 3,
R ($C_1$-$C_{14}$)-Acyl und
$R^1$ ($C_1$-$C_6$)-Alkyl, ($C_3$-$C_7$)-Cycloalkyl, ($C_7$-$C_{11}$)-Aralkyl oder eine andere Carboxylschutzgruppe bedeuten, wobei die Wasserstoffatome an den Brückenkopf-Kohlenstoffatomen 3a und (5+n)a vorzugsweise cis-konfiguriert sind, dadurch gekennzeichnet,
daß man eine Verbindung der Formel II

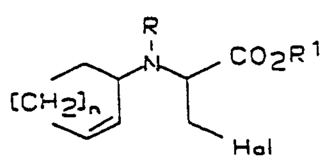

(II)

in welcher n, R und $R^1$ wie oben definiert sind und Hal Chlor, Brom oder Iod bedeutet, in Gegenwart von Trialkylstannan und gegebenenfalls eines Radikalinitiators radikalisch cyclisiert.

2. Verfahren gemäß Anspruch 1, dadurch gekennzeichnet, daß man eine Verbindung der Formel II cyclisiert, in welcher
   R ($C_1$-$C_6$)-Alkanoyl, ($C_6$-$C_{10}$)-Aryl-($C_1$-$C_4$)-alkanoyl, ($C_6$-$C_{10}$)-Aroyl, ($C_1$-$C_6$)-Alkoxycarbonyl oder ($C_7$-$C_{11}$)-Aralkyloxycarbonyl bedeutet.

3. Verfahren gemäß Anspruch 1, dadurch gekennzeichnet, daß man eine Verbindung der Formel II cyclisiert, in welcher R für eine in der Peptidchemie übliche Aminschutzgruppe vom Urethantyp steht.

4. Verfahren gemäß einem oder mehreren der Ansprüche 1 bis 3, dadurch gekennzeichnet, daß man eine Verbindung der Formel II cyclisiert, in welcher $R^1$ ($C_1$-$C_4$)-Alkyl oder ($C_7$-$C_{11}$)-Aralkyl bedeutet.

5. Verfahren gemäß einem oder mehreren der Ansprüche 1 bis 3, dadurch gekennzeichnet, daß man eine Verbindung der Formel II cyclisiert, in welcher $R^1$ für eine in der Peptidchemie übliche Carboxylschutzgruppe steht.

6. Verbindung der Formel IIa,

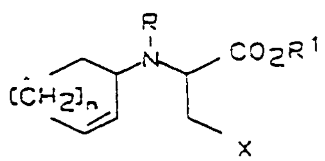

(IIa)

in welcher
n = 1, 2 oder 3,
X = Hydroxy, Chlor, Brom oder Iod,
R = Wasserstoff oder ($C_1$-$C_{14}$)-Acyl und
$R^1$ = ($C_1$-$C_6$)-Alkyl, ($C_3$-$C_7$)-Cycloalkyl, ($C_7$-$C_{11}$)-Aralkyl oder eine in der Peptidchemie übliche Carboxylschutzgruppe bedeuten.

7. Verwendung einer Verbindung der Formel IIa gemäß Anspruch 6 bei der Synthese eines ACE-Inhibitors.

8. Verfahren gemäß einem oder mehreren der Ansprüche 1-5, dadurch gekennzeichnet, daß das Trialkylstannan Tri-n-butylzinnhydrid ist.

9. Verfahren gemäß einem oder mehreren der Ansprüche 1-5 und 8, dadurch gekennzeichnet, daß der Radikalinitiator ein organisches Peroxid, z.B. Tert.- Butylperoxid, substituiertes Azobisalkansäurenitril, Mercaptan oder Stannan ist.

10. Verfahren gemäß Anspruch 9, dadurch gekennzeichnet, daß der Radikalinitiator 2,2'-Azoisobuttersäurenitril (AIBN) ist.

**Patentansprüche für folgenden Vertragsstaat : ES**

1. Verfahren zur Herstellung einer Verbindung der Formel I

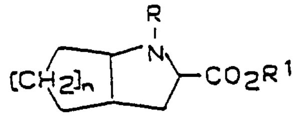

(I)

in welcher
n = 1, 2 oder 3,
R ($C_1$-$C_{14}$)-Acyl und
$R^1$ ($C_1$-$C_6$)-Alkyl, ($C_3$-$C_7$)-Cycloalkyl, ($C_7$-$C_{11}$)-Aralkyl oder eine andere Carboxylschutzgruppe bedeuten, wobei die Wasserstoffatome an den Brückenkopf-Kohlenstoffatomen 3a und (5 + n)a vorzugsweise cis-konfiguriert sind, dadurch gekennzeichnet, daß man eine Verbindung der Formel II

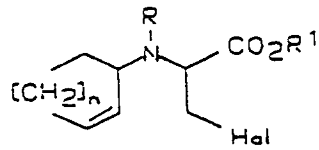

(II)

in welcher n, R und $R^1$ wie oben definiert sind und Hal Chlor, Brom oder Iod bedeutet, in Gegenwart von Trialkylstannan und gegebenenfalls eines Radikalinitiators radikalisch cyclisiert.

**2.** Verfahren gemäß Anspruch 1, dadurch gekennzeichnet, daß man eine Verbindung der Formel II cyclisiert, in welcher
R $(C_1\text{-}C_6)$-Alkanoyl, $(C_6\text{-}C_{10})$-Aryl-$(C_1\text{-}C_4)$-alkanoyl, $(C_6\text{-}C_{10})$-Aroyl, $(C_1\text{-}C_6)$-Alkoxycarbonyl oder $(C_7\text{-}C_{11})$-Aralkyloxycarbonyl bedeutet.

**3.** Verfahren gemaß Anspruch 1, dadurch gekennzeichnet, daß man eine Verbindung der Formel II cyclisiert, in welcher R für eine in der Peptidchemie übliche Aminschutzgruppe vom Urethantyp steht.

**4.** Verfahren gemäß einem oder mehreren der Ansprüche 1 bis 3, dadurch gekennzeichnet, daß man eine Verbindung der Formel II cyclisiert, in welcher $R^1$ $(C_1\text{-}C_4)$-Alkyl oder $(C_7\text{-}C_{11})$-Aralkyl bedeutet.

**5.** Verfahren gemäß einem oder mehreren der Ansprüche 1 bis 3, dadurch gekennzeichnet, daß man eine Verbindung der Formel II cyclisiert, in welcher $R^1$ für eine in der Peptidchemie übliche Carboxylschutzgruppe steht.

**6.** Verfahren zur Herstellung einer Verbindung der Formel IIa,

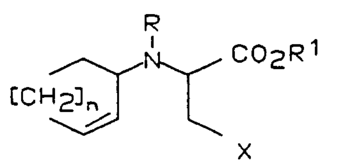

(IIa)

in welcher
n = 1, 2 oder 3,
X = Hydroxy, Chlor, Brom oder Iod,
R = Wasserstoff oder $(C_1\text{-}C_{14})$-Acyl und
$R^1$ = $(C_1\text{-}C_6)$-Alkyl, $(C_3\text{-}C_7)$-Cycloalkyl, $(C_7\text{-}C_{11})$-Aralkyl oder eine in der Peptidchemie übliche Carboxylschutzgruppe bedeuten, dadurch gekennzeichnet, daß man eine Verbindung der Formel XI

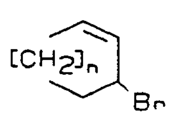

(XI)

in welcher n 1, 2 oder 3 bedeutet, umsetzt mit einem Serinester der Formel IV

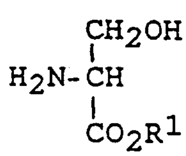

(IV)

worin $R^1$ eine veresternde Gruppe bedeutet, die entstandene Verbindung der Formel IIa, worin n und $R^1$ wie oben definiert sind, X Hydroxy und R Wasserstoff bedeuten, gegebenenfalls mit einer Verbindung der Formel Hal-R, worin Hal Halogen bedeutet und R obige Bedeutungen außer Wasserstoff hat, alkyliert, und die so erhaltene Verbindung der Formel IIa, worin n und $R^1$ wie oben definiert sind, R obige Bedeutungen außer Wasserstoff hat und X Hydroxy bedeutet, gegebenenfalls halogeniert.

**7.** Verwendung einer Verbindung der Formel IIa gemäß Anspruch 6 bei der Synthese eines ACE-Inhibitors.

**8.** Verfahren gemaß einem oder mehreren der Ansprüche 1-5, dadurch gekennzeichnet, daß das Trialkylstannan Tri-n-butylzinnhydrid ist.

**9.** Verfahren gemäß einem oder mehreren der Ansprüche 1-5 und 8, dadurch gekennzeichnet, daß der Radikalinitiator ein organisches Peroxid, z.B. tert.- Butylperoxid, substituiertes Azobisalkansäurenitril, Mercaptan oder Stannan ist.

**10.** Verfahren gemäß Anspruch 9, dadurch gekennzeichnet, daß der Radikalinitiator 2,2'-Azoisobuttersäurenitril (AIBN) ist.

## Claims

**Claims for the following Contracting States : AT, BE, CH, DE, FR, GB, GR, IT, LI, LU, NL, SE**

**1.** A process for the preparation of a compound of the formula I

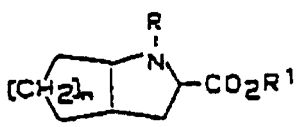

(I)

in which
n denotes 1, 2 or 3,
R denotes ($C_1$-$C_{14}$)-acyl and
$R^1$ denotes ($C_1$-$C_6$)-alkyl, ($C_3$-$C_7$)-cycloalkyl, ($C_7$-$C_{11}$)-aralkyl or another carboxyl protective group, the configuration of the hydrogen atoms at the bridge-head carbon atoms 3a and (5 + n)a preferably being cis, which comprises radical cyclization of a compound of the formula II

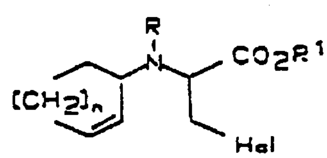

(II)

in which n, R and $R^1$ are as defined above, and Hal denotes chlorine, bromine or iodine, in the presence of trialkylstannane and with or without a free-radical initiator.

**2.** The process as claimed in claim 1, which comprises cyclizing a compound of the formula II in which R denotes ($C_1$-$C_6$)-alkanoyl, ($C_6$-$C_{10}$)-aryl-($C_1$-$C_4$)-alkanoyl, ($C_6$-$C_{10}$)-aroyl, ($C_1$-$C_6$)-alkoxycarbonyl or ($C_7$-$C_{11}$)-aralkyloxycarbonyl.

**3.** The process as claimed in claim 1, which comprises cyclizing a compound of the formula II in which R represents an one protective group of the urethane type customary in peptide chemistry.

**4.** The process as claimed in one or more of claims 1 to 3, which comprises cyclizing a compound of the formula II in which $R^1$ denotes ($C_1$-$C_4$)-alkyl or ($C_7$-$C_{11}$)-aralkyl.

5. The process as claimed in one or more of claims 1 to 3, which comprises cyclizing a compound of the formula II in which $R^1$ represents a carboxyl protective group customary in peptide chemistry.

6. A compound of the formula IIa

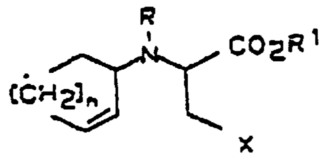

(IIa)

in which
n denotes 1, 2 or 3,
X denotes hydroxyl, chlorine, bromine or iodine,
R denotes hydrogen or $(C_1-C_{14})$-acyl, and
$R^1$ denotes $(C_1-C_6)$-alkyl, $(C_3-C_7)$-cycloalkyl, $(C_7-C_{11})$-aralkyl or a carboxyl protective group customary in peptide chemistry.

7. The use of a compound of the formula IIa as claimed in claim 6 in the synthesis of an ACE inhibitor.

8. The process as claimed in one or more of claims 1 - 5, wherein the trialkylstannane is tri-n-butyltin hydride.

9. The process as claimed in one or more of claims 1 - 5 and 8, wherein the free-radical initiator is an organic peroxide, for example tert-butyl peroxide, a substituted azobisalkanenitrile, thiol or stannane.

10. The process as claimed in claim 9, wherein the free-radical initiator is 2,2'-azobisisobutyronitrile (AIBN).

**Claims for the following Contracting State : ES**

1. A process for the preparation of a compound of the formula I

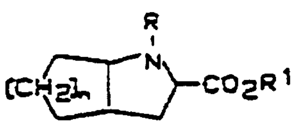

(I)

in which
n denotes 1, 2 or 3,
R denotes $(C_1-C_{14})$-acyl and
$R^1$ denotes $(C_1-C_6)$-alkyl, $(C_3-C_7)$-cycloalkyl, $(C_7-C_{11})$-aralkyl or another carboxyl protective group, the configuration of the hydrogen atoms at the bridge-head carbon atoms 3a and (5+n)a preferably being cis, which comprises radical cyclization of a compound of the formula II

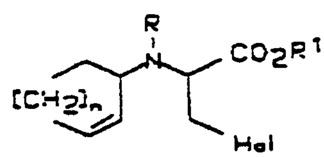

(II)

in which n, R and $R^1$ are as defined above, and Hal denotes chlorine, bromine or iodine, in the presence of trialkylstannane and with or without a free-radical initiator.

2. The process as claimed in claim 1, which comprises cyclizing a compound of the formula II in which R denotes $(C_1-C_6)$-alkanoyl, $(C_6-C_{10})$-aryl-$(C_1-C_4)$-alkanoyl, $(C_6-C_{10})$-aroyl, $(C_1-C_6)$-alkoxycarbonyl or $(C_7$-

$C_{11}$)-aralkyloxycarbonyl.

**3.** The process as claimed in claim 1, which comprises cyclizing a compound of the formula II in which R represents an amine protective group of the urethane type customary in peptide chemistry.

**4.** The process as claimed in one or more of claims 1 to 3, which comprises cyclizing a compound of the formula II in which $R^1$ denotes ($C_1$-$C_4$)-alkyl or ($C_7$-$C_{11}$)-aralkyl.

**5.** The process as claimed in one or more of claims 1 to 3, which comprises cyclizing a compound of the formula II in which $R^1$ represents a carboxyl protective group customary in peptide chemistry.

**6.** A process for the preparation of a compound of the formula IIa

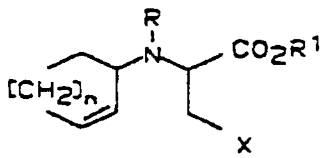

(IIa)

in which
n denotes 1, 2 or 3,
X denotes hydroxyl, chlorine, bromine or iodine,
R denotes hydrogen or ($C_1$-$C_{14}$)-acyl, and
$R^1$ denotes ($C_1$-$C_6$)-alkyl, ($C_3$-$C_7$)-cycloalkyl, ($C_7$-$C_{11}$)-aralkyl or a carboxyl protective group customary in peptide chemistry,
which comprises reacting a compound of the formula XI

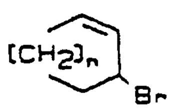

(XI)

in which n denotes 1, 2 or 3, with a serine ester of the formula IV

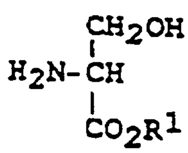

(IV)

in which $R^1$ is an esterifying group, alkylating the resulting compound of the formula IIa, in which n and $R^1$ are as defined above, X denotes hydroxyl and R denotes hydrogen, using if desired a compound of the formula Hal-R in which Hal denotes halogen and R is as defined above except for hydrogen, and halogenating if desired the resulting compound of the formula IIa, in which n and $R^1$ are as defined above, R is as defined above except for hydrogen and X denotes hydroxyl.

**7.** The use of a compound of the formula IIa as claimed in claim 6 in the synthesis of an ACE inhibitor.

**8.** The process as claimed in one or more of claims 1 - 5, wherein the trialkylstannane is tri-n-butyltin hydride.

**9.** The process as claimed in one or more of claims 1 - 5 and 8, wherein the free-radical initiator is an organic peroxide, for example tert-butyl peroxide, a substituted azobisalkanenitrile, thiol or stannane.

**10.** The process as claimed in claim 9, wherein the free-radical initiator is 2,2'-azobisisobutyronitrile (AIBN).

**Revendications**

**Revendications pour les Etats contractants suivants : AT, BE, CH, DE, FR, GB, GR, IT, LI, LU, NL, SE**

**1.** Procédé pour la préparation de composés de formule I

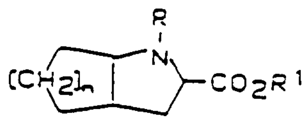

(I)

dans laquelle
n = 1, 2 ou 3,
R représente un groupe acyle en $C_1$-$C_{14}$ et
$R^1$ représente un groupe alkyle en $C_1$-$C_6$, cycloalkyle en $C_3$-$C_7$, aralkyle en $C_7$-$C_{11}$, ou un autre groupe protecteur de fonction carboxy, les atomes d'hydrogène sur les atomes de carbone en tête de pont 3a et (5 + n) étant de préférence en configuration *cis*,
caractérisé en ce que l'on soumet à une cyclisation radicalaire un composé de formule II

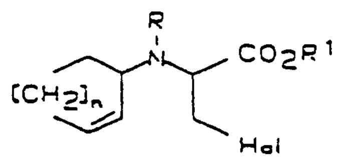

(II)

dans laquelle n, R et $R^1$ sont tels que définis plus haut et Hal représente le chlore, le brome ou l'iode, en présence d'un trialkylstannane et éventuellement d'un initiateur de radicaux libres.

**2.** Procédé selon la revendication 1, caractérisé en ce que l'on cyclise un composé de formule II dans lequel R représente un groupe alcanoyle en $C_1$-$C_6$, aryl($C_6$-$C_{10}$)-alcanoyle($C_1$-$C_4$), aroyle en $C_6$-$C_{10}$, alcoxy($C_1$-$C_6$)-carbonyle ou aralkyloxy($C_7$-$C_{11}$)-carbonyle.

**3.** Procédé selon la revendication 1, caractérisé en ce que l'on cyclise un composé de formule II dans lequel R représente un groupe protecteur de fonction amine, du type uréthanne, usuel dans la chimie des peptides.

**4.** Procédé selon une ou plusieurs des revendication 1 à 3, caractérisé en ce que l'on cyclise un composé de formule II dans lequel $R^1$ représente un groupe alkyle en $C_1$-$C_4$ ou aralkyle en $C_7$-$C_{11}$.

**5.** Procédé selon une ou plusieurs des revendication 1 à 3, caractérisé en ce que l'on cyclise un composé de formule II dans lequel $R^1$ représente un groupe protecteur de fonction carboxy, usuel dans la chimie des peptides.

**6.** Composé de formule IIa

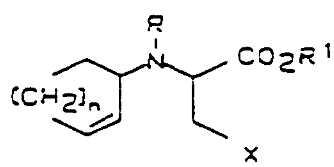

(IIa)

dans laquelle
n = 1, 2 ou 3,
X représente le groupe hydroxy ou un atome de chlore, de brome ou d'iode,
R représente un atome d'hydrogène ou un groupe acyle en $C_1$-$C_{14}$, et

$R^1$ représente un groupe alkyle en $C_1$-$C_6$, cycloalkyle en $C_3$-$C_7$, aralkyle en $C_7$-$C_{11}$, ou un groupe protecteur de fonction carboxy, usuel dans la chimie des peptides.

**7.** Utilisation d'un composé de formule IIa selon la revendication 6, dans la synthèse d'un inhibiteur de l'enzyme de conversion de l'angiotensine (ACE).

**8.** Procédé selon une ou plusieurs des revendication 1 à 5, caractérisé en ce que le trialkylstannane est l'hydrure de tri-n-butylétain.

**9.** Procédé selon une ou plusieurs des revendications 1 à 5 et 8, caractérisé en ce que l'initiateur de radicaux libres est un peroxyde organique, par exemple le peroxyde de tert-butyle, un azo-bis-alcanonitrile substitué, un mercaptan ou un stannane.

**10.** Procédé selon la revendication 9, caractérisé en ce que l'initiateur de radicaux libres est le 2,2'-azo-bisisobutyronitrile (AIBN).

**Revendications pour l'Etat contractant suivant : ES**

**1.** Procédé pour la préparation de composés de formule I

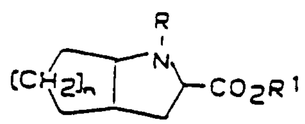

(I)

dans laquelle
- n = 1, 2 ou 3,
- R représente un groupe acyle en $C_1$-$C_{14}$ et
- $R^1$ représente un groupe alkyle en $C_1$-$C_6$, cycloalkyle en $C_3$-$C_7$, aralkyle en $C_7$-$C_{11}$, ou un autre groupe protecteur de fonction carboxy, les atomes d'hydrogène sur les atomes de carbone en tête de pont 3a et (5 + n) étant de préférence en configuration *cis*,

caractérisé en ce que l'on soumet à une cyclisation radicalaire un composé de formule II

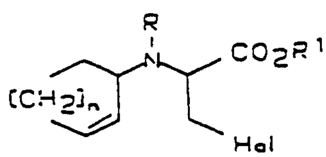

(II)

dans laquelle n, R et $R^1$ sont tels que définis ci-dessus et Hal représente le chlore, le brome ou l'iode, en présence d'un trialkylstannane et éventuellement d'un initiateur de radicaux libres.

**2.** Procédé selon la revendication 1, caractérisé en ce que l'on cyclise un composé de formule II dans lequel R représente un groupe alcanoyle en $C_1$-$C_6$, aryl($C_6$-$C_{10}$)-alcanoyle($C_1$-$C_4$), aroyle en $C_6$-$C_{10}$, alcoxy($C_1$-$C_6$)-carbonyle ou aralkyloxy($C_7$-$C_{11}$)-carbonyle.

**3.** Procédé selon la revendication 1, caractérisé en ce que l'on cyclise un composé de formule II dans lequel R représente un groupe protecteur de fonction amine, du type uréthanne, usuel dans la chimie des peptides.

**4.** Procédé selon une ou plusieurs des revendication 1 à 3, caractérisé en ce que l'on cyclise un composé de formule II dans lequel $R^1$ représente un groupe alkyle en $C_1$-$C_4$ ou aralkyle en $C_7$-$C_{11}$.

**5.** Procédé selon une ou plusieurs des revendication 1 à 3, caractérisé en ce que l'on cyclise un composé de formule II dans lequel $R^1$ représente un groupe protecteur de fonction carboxy, usuel dans la chimie des peptides.

6. Procédé pour la préparation d'un composé de formule IIa

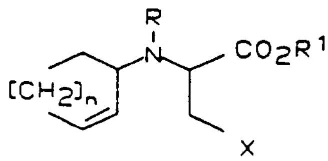

(IIa)

dans laquelle

- n = 1, 2 ou 3,
- X représente le groupe hydroxy ou un atome de chlore, de brome ou d'iode,
- R représente un atome d'hydrogène ou un groupe acyle en $C_1$-$C_{14}$, et
- $R^1$ représente un groupe alkyle en $C_1$-$C_6$, cycloalkyle en $C_3$-$C_7$, aralkyle en $C_7$-$C_{11}$, ou un groupe protecteur de fonction carboxy, usuel dans la chimie des peptides,

caractérisé en ce que l'on fait réagir un composé de formule XI

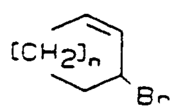

(XI)

dans laquelle n représente 1, 2 ou 3,
avec un ester de sérine de formule IV

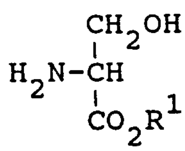

(IV)

dans laquelle $R^1$ représente un groupe estérifiant, on soumet éventuellement à une alkylation le composé de formule IIa obtenu, dans lequel n et $R^1$ sont tels que définis plus haut, X représente le groupe hydroxy et R représente un atome d'hydrogène, avec un composé de formule Hal-R dans laquelle Hal représente un halogène et R a les significations données plus haut à l'exception de celle d'un atome d'hydrogène, et on soumet éventuellement à une halogénation le composé de formule IIa ainsi obtenu, dans lequel n et $R^1$ sont tels que définis plus haut, R a les significations données plus haut à l'exception de celle d'un atome d'hydrogène, et X représente le groupe hydroxy.

7. Utilisation d'un composé de formule IIa selon la revendication 6, dans la synthèse d'un inhibiteur de l'enzyme de conversion de l'angiotensine (ACE).

8. Procédé selon une ou plusieurs des revendication 1 à 5, caractérisé en ce que le trialkylstannane est l'hydrure de tri-n-butylétain.

9. Procédé selon une ou plusieurs des revendications 1 à 5 et 8, caractérisé en ce que l'initiateur de radicaux libres est un peroxyde organique, par exemple le peroxyde de tert-butyle, un azo-bis-alcanonitrile substitué, un mercaptan ou un stannane.

10. Procédé selon la revendication 9, caractérisé en ce que l'initiateur de radicaux libres est le 2,2'-azo-bisisobutyronitrile (AIBN).